Europäisches Patentamt

(19) European Patent Office

Office européen des brevets

(11) Publication number : **0 328 542 B1**

# (12) EUROPEAN PATENT SPECIFICATION

(45) Date of publication of patent specification :
04.03.92 Bulletin 92/10

(21) Application number : 87907254.4

(22) Date of filing : 09.10.87

(86) International application number :
PCT/SE87/00456

(87) International publication number :
WO 88/03271 05.05.88 Gazette 88/10

(51) Int. Cl.⁵ : **G01N 33/53,** A61K 49/00, A61K 39/395, C12N 5/00, C07K 15/00

(54) ANTIPARATHYROID ANTIBODY PREPARATION.

(30) Priority : 28.10.86 SE 8604588

(43) Date of publication of application :
23.08.89 Bulletin 89/34

(45) Publication of the grant of the patent :
04.03.92 Bulletin 92/10

(84) Designated Contracting States :
AT BE CH DE FR GB IT LI LU NL SE

(56) References cited :
EP-A- 227 391
US-A- 4 508 828
PROCEEDINGS OF THE NATIONAL ACADEMY
OF SCIENCES OF THE USA, vol. 83, August
1986; W.G.CANCE et al., pp. 6112-6116
CHEMICAL ABSTRACTS, vol. 106, 1987, Columbus, OH (US); no. 79156u

(73) Proprietor : JUHLIN, Claes
Rälsvägen 69
S-752 52 Uppsala (SE)
Proprietor : HOLMDAHL, Rikard
Bergagatan 21
S-752 39 Uppsala (SE)

Proprietor : KLARESKOG, Lars
Ingvarsgatan 33
S-753 34 Uppsala (SE)
Proprietor : RASTAD, Jonas
Rälsvägen 50
S-752 52 Uppsala (SE)
Proprietor : AKERSTRÖM, Göran
S. Rudbecksgatan 10
S-752 36 Uppsala (SE)

(72) Inventor : JUHLIN, Claes
Rälsvägen 69
S-752 52 Uppsala (SE)
Inventor : HOLMDAHL, Rikard
Bergagatan 21
S-752 39 Uppsala (SE)
Inventor : KLARESKOG, Lars
Ingvarsgatan 33
S-753 34 Uppsala (SE)
Inventor : RASTAD, Jonas
Rälsvägen 50
S-752 52 Uppsala (SE)
Inventor : AKERSTRÖM, Göran
S. Rudbecksgatan 10
S-752 36 Uppsala (SE)

(74) Representative : Thylén, Eva
c/o Pharmacia LEO Therapeutics AB Box 941
S-251 09 Helsingborg (SE)

## Description

This invention is concerned with antibody preparations which are useful in the diagnosis and treatment of parathyroid and renal disorders.

The term "preparation" is used in order to emphasize that the specificity of the antibody preparations of the invention has been limited in some way or other with respect to the antigenic determinants involved. Thus the term "antibody preparation" according to the invention does not comprise any such instances where the antibodies exist potentially' in vivo or coexist in vitro with other antibodies directed against determinants which are not unique to parathyroid and/or kidney tissue.

The frequency of diagnosed cases of primary hyperparathyroidism (HPT) is increasing all over the world, and consequently the number of parathyroid operations is also increasing. Reoperations are not common but in the case of HPT they will severely affect the patient, inasmuch as the scar tissue formed will considerably thwart anatomical dissection. It has been shown earlier that an adequate preoperative localization of parathyroid glands will produce improved results in reoperations of HPT patients.

For the preoperative localization of enlarged parathyroid glands the techniques employed nowadays are mostly of the non-invasive type, such as e.g. ultrasonography and CT scanning, but the result of the examination is clearly dependent on the size of the gland, it being often impossible to localize glands that are small or have become enlarged to only a moderate degree. Glands in retroesophageal, retroclavicular and mediastinal positions cannot be detected by ultrasound techniques, and hyperplastic glands are seen to only a minor extent. Magnetic resonance tomography (MRT) has recently been introduced as a tool for preoperative localization diagnostics in cases of parathyroid disorders. The diagnostic results obtained with this type of examination are at present being evaluated, but it appears that MRT, as well as CT and invasive methods previously employed for localization diagnostics are hampered by similar and sometimes even greater difficulties as compared to ultrasonography. Evidently the methods currently available are not capable of visualizing small parathyroid glands in atypical positions, i.e. those that are the most difficult to be located by the surgeon. Moreover there is the problem of establishing the identity of an ultrasonically detected alternation; a mistake easily made is that e.g. thyroid nodules and lymph nodes are wrongly interpreted as being parathyroid glands. Therefore, in order to minimize the risk of a wrong diagnosis a directed fine needle puncture technique is sometimes resorted to as a complement to ultrasound and CT examinations. But the evaluation of the puncture samples presents some difficulties, in particular as regards discrimination of parathyroid cells from thyroid epithelium, because adequate differential diagnostic staining methods are not yet available.

Several diagnostic problems in the field of parathyroid diseases would be solved by antibody preparations directed against surface antigens that are unique to parathyroid cells. It would be possible to employ such preparations for preoperatively localizing parathyroid glands in vivo. Moreover such preparations could be utilized in conjunction with immunocytochemical techniques as a means to improve the differential diagnostic evaluation of samples obtained by fine needle puncturing, and also as a tool in histopathological diagnostics of adenomatous and hyperplastic parathyroid glands. Other fields of use that may be mentioned are for example detection and quantification of potentially released forms of the said type of unique antigens, e.g. in whole blood, serum, plasma and urine samples. An antibody preparation directed against a cell surface antigen on parathyroid cells has been described recently (1). This preparation, in contrast to that of the invention, has been found to be entirely negative in its reaction with renal tissue. Such a preparation therefore can never cover such a wide field of use as the antibody preparations of the present invention. However, a patent application has been directed against it (12).

We have now discovered that parathyroid cells express unique cell surface antigens having determinants which can be immunologically identified also in certain regions of the human kidney, more precisely on cells in proximal tubuli and in the parietal layer of Bowman's capsule. We have also unambiguously shown that such unique surface antigens may be of paramount importance for the calcium metabolism of the parathyroid cells. These surface antigens may correspond to one calcium receptor/channel. The occurrence of such a receptor on parathyroid cells has been discussed earlier, but its existence has not been proved conclusively (2). If a cell surface antigen is "unique" in the sense as set forth above this of course does not exclude the possibility that minute amounts of the same antigen may be detected some time in the future also in other places.

The invention comprises antibody preparations the specificity of which is unique to the aforesaid cell surface antigens. The preparation thus has antibody activity against parathyroid cells and certain renal cells but not against other tissues such as those set forth in the exemplification (see Table 1). As regards the epithelial cells of proximal tubuli, the antibodies and antibody preparations of the invention are reactive preferentially with cell portions facing the interior of the tubuli (brush border) as compared to cell portions facing towards the opposite side (luminal border). Reactive cells will be found all the way up to Bowman's capsule, so the antigen is detectable also in cells of the parietal layer of a number of kidney glomeruli. No reactivity has been demonstrable in epithelial cells lining the interior of the glomeruli (visceral cells) or in distal tubuli. In the preferred embodiment, all the antiparathyroid antibody-active components of the preparation react with these unique antigenic determinants.

During the priority year we have found that our antibody preparations also react with placenta (cytotrophoblastic cells). This is in consistency with the known fact that an active $Ca^{2+}$ transport is at hand across the placenta. Accordingly, the antibody preparation of the invention is also reactive with placenta tissue.

Antibody preparations possessing specificity for various renal antigens have been described earlier (see for example 3, 4, 5). However, none of these preparations have been directed specifically against the same antigenic determinants as the preparations of this invention. To facilitate an evaluation of the patentability of our antibody preparations we will now discuss in some detail those known monoclonals which we believe might be confused with ours. Among the TN1-TN10 antibodies, only TN1 and TN10 are reactive with antigens that are unique to distinct renal cells (4). TN1 reacts with the brush border portion of epithelial cells in proximal tubuli, but differs from the antibody preparations of this invention in that its reactivity is directed aginst an intracellular component. No reactivity at all has been found to occur with epithelial cells of the parietal layer. As regards TN10, it is true that this antibody is specific for a cell surface antigen, but such specificity has been demonstrable only in respect of visceral epithelial cells of glomeruli. Epithelial cells in the parietal layer and in proximal tubuli have been negative vis-à-vis TN10. The AJ8 antibody, while indeed being directed against an antigenic determinant expressed on epithelial cells of proximal tubuli and glomeruli (3), nevertheless cannot be said to be directed against a uniquely renal antigen inasmuch as the said determinant will be found also on melanocytes and fibroblasts (6, esp. Table 3 page 176). The antibody $S_4$ identifies a 160 kD glycoprotein which is expressed in glomeruli generally and in proximal tubuli (3, 5).

The antibody preparation of the invention may be in the form of a nomoclonal antibody or a polyclonal antibody (antiserum). The antibodies present in the preparation may be fragmented and/or derivatized. The important point to be heeded here is that the fragments and/or derivatives have to possess biospecific immune-type affinity, with the above-mentioned specificity and cross reactivity. The preparation may be in the form of a solution with various additions of chemicals as required for a particular use, e.g. buffer systems and detergents. What is important is that the preparation must not contain other components in amounts that would interfere with the actual use contemplated. In its preferred embodiment the preparation is monoclonal; optionally it may be a mixture of a finite number of different monoclonal antibody-active components. It is preferred that the antibodies of the preparation be of the IgG type.

As regards various derivatized forms of the antibodies according to the invention, forms worthwhile to be mentioned in addition to the fragments Fab, Fab' and $F(ab')_2$, are such in which the intact antibody or one of the said fragments is covalently bound to an analytically detectable group such as a radioactive, fluorescent, chemiluminescent, enzymatically active, biotinyl etc. group. Antibody-active components may also be covalently or adsorptively bound to various so-called solid phases, that is, phases which are insoluble in aqueous media. In certain embodiments, the antibody-active components of the preparation may be bound to a drug such as e.g. a cytotoxic drug.

Production of an antibody preparation according to the invention is performed in that cells potentially capable of producing antibodies that possess a specificity in accordance with the invention are caused to excrete said antibodies, whereupon the antibodies thus excreted are isolated and purified so as to remove those antibodies that do not fulfil the specificity requirements. In a vertebrate (e.g. mammal such as mouse or rat) the said excretion may take place in vivo as a result of immunization with a suitable immunogen. The immune response thus obtained will give a polyclonal antibody preparation containing the desired antibody/antibodies in admixture with antibodies directed against other determinants in the immunogen. For the removal of these latter antibodies recourse may be had to so-called immunosorbent purification (IS purification). In the case of the antibodies as contemplated here, IS purification will produce low yields and involve laborious working procedures but will very probably not acquire any practical importance before the antigen has been isolated.

The best method of obtaining a good antibody preparation according to the invention is by way of a so-called monoclonal technique. This is carried out in that after induction of immunization the antibody-producing cells are fused with myeloma cells so as to enable them to grow quickly and incessantly. By cloning and culturing the hybrid cells which produce antibodies of a specificity as according to the present invention it is possible to produce monoclonal antibody preparations reacting in principle only with the desired determinants in parathyroid and renal tissues. Culturing of the selected cell clones for the production of the antibody preparations of this invention may be performed in cell cultures in vitro or as ascites tumors. Purification and isolation may be carried out in the same manner as in common practice for antibodies in general by salt precipitation or by means of various chromatographic methods such as ion exchange, affinity, gel etc. chromatography.

In order that the antibodies according to the invention may be obtained in an efficient manner it is preferred that the methodology of Holmdahl et al. (7) be used as applied to whole cell immunogens. A characteristic feature of that methodology is that the regional lymph nodes are externalized at a time interval of a predetermined number of days after immunization has taken place (e.g. 9 days) whereupon the B cells of the glands are fused with a suitable mouse myeloma cell line.

The antibody preparation of the invention can be used for forming immune complexes containing the homologous antigen against which the antibodies of the invention are directed. Immune complex formation is an essential part of many technical applications of immunological procedures, for instance in the contexts of affinity purification (e.g. immunosorbent purification) and of immunochemical assays for qualitatively or quantitatively demonstrating the presence of the antigen both in vivo and in vitro. The practical measures to be taken comprise administration of the preparations according to the invention to a test object (including live mammals, e.g. humans) containing the homologous antigen against which the preparation is directed, so that the complex can be formed. The reaction conditions employed are those commonly relied on in the case of each respective field of application. Thus for in vitro work the pH and temperature should be chosen within the range of 5-9 and the range of +4 - +40 °C respectively, and suitable buffer systems and detergents may be added. Numerous immunoassay methods are available; it is up to the skilled artisan to decide in each case which method should be chosen as being the most suitable. Among various-general methods may be mentioned homogeneous and heterogeneous methods, so-called sandwich methods, double antibody methods (for example DASP), competitive (inhibitions) and non-competitive methods, particle agglutination, precipitation methods, immunoelectrophoresis, immunodiffusion, histochemical and cytochemical methods, microscopy with the aid of labeled antibodies, and methods named according to their various marker systems, like radio-, enzyme-, fluorescence-, chemiluminescence-, enzyme substrate-, biotinyl-immunochemical methods. Thus tissues and cells which express the antigens contemplated may be studied immunochemically in a manner known per se but using the preparations of the present invention. In particular, the staining pattern obtained in cases of parathyroid and renal cancers is clearly distinguishable from that of the corresponding normal tissues. The same is also true for parathyroid adenoma and primary and secondary hyperplasma compared to normal parathyroid tissue. The preparations can be employed also for distinguishing the cell types contemplated from other cells in a cell sample, and for scintigraphically locating parathyroid glands in vivo.

Protein preparations rich in the antigenic protein or fragments thereof react with the antibody active components of an antibody preparation of the invention. These latter protein preparations can be produced as described in the experimental portion of these specifications. With respect to proteins originating from the source of raw material in these preparations, the protein reacting with the monoclonal antibodies described in this specification is substantially pure, i.e. mostly amounts to more than 90 % (w/w) of proteins from the source of raw material.

The invention is further defined in the dependent claims which form a part of this specification. The invention will now be illustrated by way of the corresponding scientific work.

EXPERIMENTAL PORTION

MATERIALS AND METHODS

Biopsies and tissue preparations: Biopsies were obtained from normal human parathyroid glands removed during thyroid operations and from normal and adenomatous glands taken from patients who were undergoing surgery for hyperparathyroidism. Other human tissue samples were obtained from organs removed during routine surgery procedures or from organs taken for transplantation purposes. Parathyroid biopsies and various tissue samples were also obtained from calves, monkeys and rats. Tissues to be used for immunohistochemistry were placed in ice-cold Histocon (Histolab, Bethlehem Trading Ltd, Gothenburg, Sweden). Dispersed cells were prepared from parathyroid adenoma by collagenase treatment and purification on Percoll (Pharmacia AB, Sweden) as previously described (8). Live cells were frozen and preserved in liquid nitrogen. The cells upon thawing showed the same viability and response to external $Ca^{2+}$ as freshly prepared cells (see below).

Immunization and hybridoma production: 50 µl of dispersed parathyroid adenoma cells ($0.5 \times 10^6$ cells/ml) were suspended after thawing in an equal amount of Freund's complete adjuvant (Difco, Detroit, USA) and used to immunize 5 male DBA/1 mice. Following a recently described protocol for hybridoma production (7), popliteal (knee) and inguinal (groin) lymph nodes were removed 9 days after immunization and were used for fusion with mouse myeloma cells (cell line X-63-653XAg8). After fusion the hybridomas were incubated with a HAT-containing medium in microtiter wells and screened for growing hybridomas by phase contrast microscopy. In a first immunohistochemical screening operation, hybridomas were identified which produced antibodies reactive with parathyroid cells. Positive hybridomas were subcloned using limiting dilution (0.5 cells/well), and the resultant subclones were again screened immunohistochemically, One subclone from each of the hybridomas whose supernatants were specifically reactive with surface structures of parathyroid cells was then expanded for large scale antibody production in vitro.

Immunohistochemistry and immunofluorescence: For checking up on the specificity of the hybridomas for parathyroid cells immunohistochemical stainings were performed on acetone fixed, 5 µm thick, frozen sections of various tissues. The method employed was of the PAP type (peroxidase-antiperoxidase (9)). Primary antibodies employed were either hybridoma supernatants diluted 1:5 or purified monoclonal antibodies in concentrations of 1-5 µg/ml (dissolved in PBS). Monoclonal anti-HLA-DR antibodies (Becton-Dickinson, Sunnyvale, Ca, USA) were used as positive controls, and monoclonal anticollagen II antibody (10) was used as negative control. The first immunohistochemical screening involved tests for reactivity with normal human parathyroid, thyroid and lymph nodes. Antibodies which showed exclusive positive reaction with surface structures of parathyroid parenchymal cells, i.e. were parathyroid specific, were subcloned and subsequently tested for reactivity with other human and animal tissues. Immunofluorescence stainings were performed in viable dispersed parathyroid adenoma cells and on leucocytes obtained from healthy individuals after gradient centrifugation of peripheral blood on Ficoll isopaque gradients (Pharmacia AB, Sweden). The primary antibodies used were the purified parathyroid specific antibodies (see below) in concentrations of 1-10 µg/ml, a monoclonal antibody directed against the human T-cell receptor anti-Leu 4 (Becton-Dickinson, Sunnyvale, Ca, USA), and an irrelevant anticollagen II monoclonal antibody (10). Fluorescein-labeled swine antimouse IgG antibody was employed as the secondary antibody (reactive with heavy and light chains, Swedish National Bacteriology Laboratories SBL, Sweden).

Isotype determinations and antibody purification: Isotypes of the parathyroid specific antibodies were determined by means of an ELISA method employing peroxidase-conjugated isotype specific goat antibody (Nordic Laboratories, Tilburg, Holland) and alkaline phosphatase-conjugated monoclonal rat antibody, both of these antibodies being specific for k-chains. Purification of the parathyroid specific antibodies was carried out using affinity chromatography on Protein-A-Sepharose (Pharmacia AB, Sweden) at pH 8.0 and elution at the pH recommended for each respective isotype.

Effects on cytoplasmic calcium: Dispersed parathyroid cells that had been obtained from collagenase-treated parathyroid biopsies taken as set forth above were used for studying the effects of the antibodies on cytoplasmic $Ca^{2+}$ ( $= Ca_i^{2+}$). These studies were carried out by means of microfluorimetrically recording individual cells loaded with fura-2(2). In the experiment performed, the parathyroid cells were allowed to attach to cover slips in a culture chamber accommodated in an inverted Nikon Diaphot microscope (Nikon, Japan); the microscope was equipped for microfluorometry with excitation wavelengths of 340 and 360 nm (emission 470 nm). $Ca_i^{2+}$ was monitored at external calcium concentrations of 0.5-3.0 mM with and without addition of the parathyroid specific antibody, the irrelevant anticollagen II antibody and the anti-HLA-antibody respectively.

Antigen detection in urine

The antigen (antibody binding molecule) has been measured using an ELISA (Enzyme Linked Immunosorbent Assay) as follows: Microtiterplates (Dynatech) were coated with the E11 antibody (10 µg/ml/well, overnight, 4 °C). Urine (whole, sediment or supernatant) was then added in a volume of 100 µl/well and allowed to stand in room temperature for 2 hrs, whereafter biotinylated antibody G11 was added (5 µg/ml/well) and left to react in room temperature for 2 hrs. Avidine conjugated alkaline phosphatase (1:2000) was added and allowed to stand for 2 hrs at room temperature, whereupon N-hydroxysuccidimidylbiotin (Sigma) dissolved in diethanolamine buffer (10 %, pH 9,8) was added. The colour reaction was measured at 405 nm with a Titertek Multiskan.

Characterization of the renal antibody binding molecule

The method employed has been described by Rubin K et al (11). Part of a normal human kidney removed during surgery for a localized renal cell carcinoma was frozen and stored at -20 °C. 30 grams of the thawed renal cortex was minced and homogenized in a microsomal buffer. The homogenate was passed through several layers of gauze bandage and centrifuged first at 10 000 rpm in GSA and then twice for 1 hr 100 000 x g. The pellet was homogenized in a solubilising buffer and centrifuged for 1 hr at 100 000 x g. The supernatant was applied to a Lectine Sepharose® column (Lens Culinaris Pharmacia) and the binding proteins eluted and pooled. The pooled protein fractions were then examined and found reactive in the ELISA (see above). The pooled protein fraction was then applied to a recirculating system consisting of two immunoaffinity Sepharose 4B columns (Pharmacia), one containing rabbit IgG (pre-immune) and the other the antibodies E11, G11 and B6 (28 mg antibody). The protein adsorbed in the second column was eluted, pooled and tested in the ELISA. SDS-PAGE was performed on a PhastGel gradient 10-15 (Pharmacia) utilizing the PhastSystem (Pharmacia). Prior to application samples were dialyzed against water and treated with ethanol at -20°C. The samples were reduced with beta-mercaptoethanol (final concentration 10 %) and boiled for 3 minutes. The gels were stained with Coomassie Brilliant Blue R-250. Apparent molecular weights were calculated from the migration relative the following standard proteins (Pharmacia): Bovine serum albumin (69 000), chicken ovalbumin (43 000), bovine erythrocyte carbonic anhydrase (30 000), soybean trypsin inhibitor (20 100) and bovine milk-lactalbumin (14 400).

RESULTS

Antibody specificity: Immunization with dispersed human parathyroid cells resulted in 88 proliferating hybridomas. The supernatants of 19 of these hybridomas reacted immunohistochemically with normal human parathyroids. Of these, 14 were found to exclusively stain the parenchymal cells of the gland, the others being localized in the stroma cells of the gland. Twelve of the aforesaid 14 antibodies reacted with the surface antigen of the parenchyma cells, the remaining two showing a more even staining pattern suggesting the recognition of cytoplasmic structures as well. Of the hybridomas yielding antibodies capable of staining surface structures of parathyroid epithelial cells, four were unreactive with sections of normal human thyroids and normal human lymph nodes. (These four hybridomas were named E11, G11, B6 and E5 respectively.)

Subcloning of these four parathyroid specific hybridomas did not alter the specificity of the excreted antibodies.

Immunofluorescence studies showed that the antibodies E11, G11, B6 and E5 gave rise to distinct staining of the surfaces of the dispersed parathyroid cells. These antibodies did not react with normal human leucocytes used as controls whereas the anti-Leu 4 antibody showed strong reaction with approximately 70 % of the leucocytes but did not stain the parathyroid cells. Anticollagen antibody employed as a control did not react with either one of the two types of cells. The subcloned parathyroid specific antibodies (E11, G11, B6 and E5) were studied also in respect of their reactivities with a large number of other types of tissues. Table 1 shows that all of these four antibodies bound also to epithelial cells of proximal tubuli, to the parietal layer of Bowman's capsule on several glomeruli of one and the same kidney and to cytotrophoblastic cells of placenta, and that they moreover reacted also with parathyroid and renal cancer but were entirely unreactive with the other types of tissues studied. The E11 antibody was the best stainer. The staining pattern of renal and parathyroid cancer in combination with routine-type histopathological staining differed sharply from the pattern obtained with the corresponding normal tissue. Parathyroid adenoma and primary and secondary hyperplasia had a decreased staining compared to normal parathyroid tissue. Their reactivity with certain choriocarcinoma cell lines (see Table 1) indicates a use for treating and diagnosing choroicarcinoma.

It is particularly interesting to note that the E5 hybridoma gives an IgM antibody reacting distinctly with the renal cells contemplated. Thus E5 differs clearly from the previously known IgM antibody which has been said to be parathyroid specific (1).

Purification and monoclonal isotype determination: The isotypes of the parathyroid specific antibodies were determined to be IgG 1 in the case of E11, IgG 2B in the cases of G11 and B6, and IgM in the case of E5. All these four had k-type light chains. The three IgG antibodies could all be purified by means of affinity chromatography on Protein A - Sepharose without losing their exclusive reactivity with normal renal and parathyroid cells.

Effect on cytoplasmic calcium: None of the monoclonal antibodies affected $Ca_i^{2+}$ at 0.5 mM extracellular $Ca^{2+}$, but two of them, B6 and G11 (both of the IgG $_{2b}$ type), gave rise to a complete and concentration-dependent abolishment of the $Ca_i^{2+}$ increase normally obtained when extracellular $Ca^{2+}$ is increased from 0.5 mM to 3.0 mM. The third parathyroidreactive antibody (IgG$_1$ type) and the irrelevant monoclonal IgG anticollagen II antibody did not give this effect. When the antibodies were added to the dispersed parathyroid cells after the increase of extracellular $Ca^{2+}$ to 3.0 mM the $Ca_i^{2+}$ was decreased slightly by both B6 and G11 antibodies. Here again it could be noted that neither the third antiparathyroid specific antibody (E11) nor the irrelevant anticollagen II antibody could product any effect. The fact that one of the three antiparathyroid specific antibodies did not interfere with $Ca_i^{2+}$ regulation showed that the phenomenon as ascertained by measurements was specifically characteristic of the other two antibodies. Viability tests performed after the incubations showed that the effect observed was not the result of a cytotoxic effect of the antibodies.

Antigen detection

High titres of the renal antigen has been found in the urine in connection with rejection of renal transplantations and in patients with renal insufficiences due to hypovolemic shock.

Characterization of the renal antibody binding molecule

Both the reduced and unreduced sample showed bands at 60 000 dalton and a weaker band at 20 000 dalton. The control pre-immune eluted samples gave no bands on the gel.

Discussion

Our efforts to produce monoclonal antibodies specific for parathyroid cells have been based on two assumptions: (1) Parathyroid cells due to their very special function possess a limited number of unique cell surface structures; and (2) such putative structures may be involved in functions that are unique to the cells. Our results presented in this patent application corroborate both of these assumptions. Concerning the first of these issues our data demonstrate that there is at least one set of immunogenic structures which may be unique to certain renal cells and to parathyroid cells. The observation that the four parathyroid specific antibodies showed the same tissue reactivity pattern may indicate that they are all able to recognize the same molecule(s). Consequently such molecule(s) may be assumed to be among the very few immunogenic molecules unique and common to parathyroid cells and certain renal cells. Since two of the monoclonals were capable of actively affecting the regulation of cytoplasmic $Ca^{2+}$ there is very good reason to assume that they recognize molecules involved in a $Ca^{2+}$-sensing mechanism which is unique to parathyroid and renal tubular cells. It is highly probable that the phenomenon observed is specific, in view of the fact that two of the parathyroid specific antibodies and the irrelevant anticollagen II antibody could not give a similar effect.

Our results show that the antibody preparation of this invention is an important auxiliary tool in studies of parathyroid and kidney pathophysiology, and furthermore for cytological and histopathological identifications of parathyroid and renal tissues and as an aid in preoperative scintigraphic localizations of the same tissues in vivo. Potentially the preparations may be used in the treatment of patients suffering from renal and/or parathyroid cancer, and in the diagnostication of disorders affecting the structures as here contemplated, e.g. renal tubuli. In so far as such disorders will give rise to the release of antigenic homologous molecules corresponding to the antibodies of the present invention it is not inconceivable that such diagnostication procedures may also comprise assays for these antigens in body fluids, especially urine.

TABLE 1

Immune reactivity in organs and tumors with antibodies E11, G11, B6 and E5.

Unreactive human tissues and cells: Thyroid (normal, diffuse, toxic, multinodular, atoxic), thymus, lymph nodes, lung, liver, spleen, skin, muscle, pancreas, small intestine, prostate, salivary glands, adrenal glands, bone, breast, medullary thyroid cancer, pancreatic insulinoma, midgut carcinoid tumor, Conn's tumor, Cushing's tumor, pheochromocytoma, leucocytes and sarcoma cell line.

Human tissues showing reactivity: Parathyroid, kidney, placenta (cytotrophoblastic cells), renal cell carcinoma and parathyroid cancer. In particular it was observed that the cells reacting were the epithelial cells of proximal tubuli and in the parietal layer of Bowman's capsule in a number of glomeruli of one and the same kidney. In proximal tubuli, the brush border of the cells was stained more efficiently than the luminal border. Distal tubuli were not stained at all.

Unreactive simian tissue (from Cynomolgus): Thyroid, thymus, lymph node, lung, liver, spleen, ovary and small intestine.

Simian tissue (from Cynomolgus) showing reactivity: Parathyroid and kidney.

Unreactive rat tissue: Thyroid, thymus, lymph node, lung, liver, spleen, heart, pancreas, stomach, small intestine, cerebral cortex, hypothalamus and pancreas.

Rat tissue showing reactivity: Parathyroid and kidney.

Cell line reactivity: Choriocarcinoma cell lines (JEG and JAG).

REFERENCE
- - - - - - - - -

1.   Cance, W.G. et al., Proc. Natl. Acad. Sci., USA 83 (1986) 6112-6.

2.   Gylfe, E. et al., FEBS 205 (1981) 132-6.

3.   Bander, N. H. et al., Contr. Oncol. 19 (1984) 105-20.

4.   Müller, G.A. et al., Klin. Wochenschr 61 (1983) 893-902.

5.   Ueda, R. et al., Proc. Natl. Acad. Sci., USA 78 (1981) 5122-6.

6.   Lloyd, K. O., et al., In: Basic and Clinical Immunology. Ed: Herberman, R. B., Martinus Nij Hoft Publishers (1983) 159-214 spec. page 167.

7.   Holmdahl, R., et al., J. Immunol. Meth. 83 (1985) 379-84.

8.   Rudberg, C. et al., Acta. Path. Microbiol. Immunol. Scand. Sect. A 94 (1986) 253-261.

9.   Klareskog, L. et al., Scand. J. Immunol. 16 (1982) 501-7.

10.   Holmdahl, R. et al., Arthritis and Rheumatism 29 (1986) 400-10.

11.   Rubin, K. et al., Exp. Cell Res. 164 (1986) 127-38.

12.   Washington University School of Medicine (Cance et al.) EP-A-227,391.

## Claims

1. An anti-parathyroid antibody, fragment and/or derivative thereof characterized in that the antibody active component being directed specifically against a surface antigen which in humans is unique and common to parathyroid and proximal tubuli of the kidney.

2. An anti-parathyroid antibody, fragment and/or derivative thereof according to claim 1, characterized in that the surface antigen when present in the parathyroid is wholly or partially involved in $Ca^{2+}$- sensing.

3. An anti-parathyroid antibody according to claim 1 or 2, characterized by being monoclonal.

4. An anti-parathyroid antibody, fragment and/or derivative thereof according to any of claims 1 - 3, characterized in that the antibody activ component preferably reacts with cells which are located on the inside of tubuli (brush border), as compared with those which are located on the opposite side (luminal border).

## Patentansprüche

1. Auf die Nebenschilddrüse wirkendes Antikörperfragment und/oder Derivat desselben, dadurch gekennzeichnet, dass der aktive Bestandteil des Antikörpers insbesondere gegen ein Oberflächenantigen gerichtet ist, das ausschliesslich beim Menschen vorkommt, und das die Nebenschilddrüse und die proximalen Nierenkanälchen gemeinsam haben.

2. Auf die Nebenschilddrüse wirkendes Antikörperfragment und/oder Derivat desselben, dadurch gekennzeichnet, dass das Oberflächenantigen, wenn es in der Nebenschilddrüse vorhanden ist, ganz oder teilweise an der $Ca^{2+}$-Erkennung beteiligt ist.

3. Auf die Nebenschilddrüse wirkender Antikörper nach Anspruch 1 oder 2, dadurch gekennzeichnet, dass er monoklonal ist.

4. Auf die Nebenschilddrüse wirkendes Antikörperfragment und/oder Derivat desselben nach einem der Ansprüche 1 bis 3, dadurch gekennzeichnet, dass der aktive Antikörperbestandteil vorzugsweise mit Zellen reagiert, die an der Innenseite der Kanälchen (Bürstengrenze) liegen, im Gegensatz zu jenen, die an der gegenüberliegenden Seite (Lumengrenze) liegen.

## Revendications

1. Anticorps antiparathyroïde, fragment et/ou dérivé de celui-ci, caractérisé en ce que le composant actif de l'anticorps est dirigé spécifiquement contre un antigène de surface qui chez les humains est unique et commun à la parathyroïde et aux tubules proximaux du rein.

2. Anticorps antiparathyroïde, fragment et/ou dérivé de celui-ci selon la revendication 1, caractérisé en ce que l'antigène de surface lorsqu'il est présent dans la parathyroïde est entièrement ou partiellement impliqué dans la détection du $Ca^{2+}$.

3. Anticorps antiparathyroïde selon la revendication 1 ou 2, caractérisé par le fait d'être monoclonal.

4. Anticorps antiparathyroïde, fragment et/ou dérivé de celui-ci selon l'une quelconque des revendications 1 - 3, caractérisé en ce que le composant actif de l'anticorps réagit de préférence avec des cellules qui sont situées à l'intérieur des tubules (bords en brosse) par rapport à celles qui sont situées sur le côté opposé (bord luminal).